# EUROPEAN PATENT APPLICATION

(11) **EP 4 760 239 A1**
(43) Date of publication of application: **17.06.2026**
(21) Application number: 25215560.1
(22) Date of filing: 13.11.2025
(51) Int. Cl.: G01N 21/35, G01N 21/65, G01N 21/88, G01N 21/3581, G01N 33/28

(54) **METHOD OF PERFORMING SPECTRAL ANALYSIS**

(30) Priority: 13.12.2024 GB 202418313
(71) Applicant: Rolls-Royce plc, London N1 9FX (GB)
(72) Inventor: Gameros Madrigal, Andres A, Derby, DE24 8BJ (GB)
(74) Representative: Rolls-Royce plc

(57) **Abstract**

A method (100) of performing spectral analysis on a sample (220) identified within a gas turbine engine (10). The method (100) involves: obtaining (102) an optical spectrum (240) of the sample (220); normalising (104) the optical spectrum (240) to obtain a normalised optical spectrum (301); selecting (106) a characteristic parameter that characterises the normalised optical spectrum (301); determining (108) a characteristic parameter value associated with the normalised optical spectrum (301) based on the characteristic parameter; comparing (110) the characteristic parameter value with one or more predetermined reference values corresponding to the characteristic parameter; and determining (112) a presence of at least one compound in the sample (220) based on the comparison between the characteristic parameter value and the one or more predetermined reference values. The method can determine whether there is a need to disassemble the gas turbine engine for maintenance.

## Description

### FIELD

This invention relates to a method of performing spectral analysis. More particularly, this invention relates to a method of performing spectral analysis on a sample identified within a gas turbine engine.

### BACKGROUND

During inspection of a gas turbine engine, various substances may be visually identified within the gas turbine engine. It may be important to detect the substances, or more specifically, the compounds in the substances, to follow a suitable maintenance procedure for the gas turbine engine. As an example, a smear of liquid detected within the gas turbine engine may be caused by a spillage of an inhibitor fluid (e.g., a corrosion inhibitor fluid), or by a leakage of an engine oil. If the smear is caused by the spillage of the inhibitor fluid, the maintenance may continue without further stripping of the gas turbine engine. In contrast, if the smear is caused by the leakage of the engine oil, the gas turbine engine may need to be stripped further to investigate the cause of the smear, which might be a time consuming and expensive operation. Identification of the compounds of the smear may be important to ensure that engine strips are performed only when necessary.

Spectroscopic techniques are widely used in the qualitative and quantitative analysis of chemical constitution of various organic and inorganics. For example, Raman spectroscopy is a non-destructive technique for analysing the composition of a substance. Conventional spectral analysis techniques may include comparing the location, shape, and intensity of peaks obtained in a Raman spectrum to a reference library in order to identify the composition of the substance. However, such conventional spectral analysis techniques may have some drawbacks. Firstly, such techniques may require computationally complex pre-processing (e.g., deconvolution) so that these peaks can be easily identified for analysis. Furthermore, in some cases, these peaks may be masked altogether due to a fluorescence effect. Moreover, in some cases, the quality of the signal from a spectroscope may not be strong enough to reveal these peaks.

### SUMMARY

In a first aspect, there is provided a method of performing spectral analysis on a sample identified within a gas turbine engine. The method includes obtaining an optical spectrum of the sample. The method further includes normalising the optical spectrum to obtain a normalised optical spectrum. The method further includes selecting a characteristic parameter that characterises the normalised optical spectrum. The method further includes determining a characteristic parameter value associated with the normalised optical spectrum based on the characteristic parameter. The method further includes comparing the characteristic parameter value with one or more pre-determined reference values corresponding to the characteristic parameter. The method further includes determining a presence of at least one compound in the sample based on the comparison between the characteristic parameter value and the one or more pre-determined reference values.

The method may facilitate determining the at least one compound in the sample in a computationally efficient manner. The method may utilise the optical spectrum without performing computationally expensive pre-processing operations (e.g., deconvolution, fluorescence removal, etc.) on the optical spectrum, which are typically performed in conventional spectral analysis techniques. The method may be conveniently performed, for example, using edge computing devices, such as handheld computing devices near the gas turbine engine due to the low computational complexity compared to conventional spectral analysis techniques. Moreover, the method may utilise the fluorescence background in the optical spectrum to determine the at least one compound, in contrast to conventional spectral analysis techniques that consider fluorescence as noise.

The method may also facilitate inspection and maintenance of the gas turbine engine. For example, the method may simplify decision-making during a maintenance procedure based on the determined presence of the at least one compound in the sample. By way of example, the method may facilitate a decision to further strip the gas turbine engine or continue inspection without further stripping the gas turbine engine.

In some embodiments, the method further includes determining a region of interest across the optical spectrum, and normalising the optical spectrum includes normalising the optical spectrum defined in the region of interest.

In some embodiments, the region of interest extends from a first wavenumber to a second wavenumber that is greater than the first wavenumber.

In some embodiments, the method further includes processing the optical spectrum of the sample prior to normalising the optical spectrum. The processing is devoid of deconvolution processing.

In some embodiments, the processing of the optical spectrum of the sample is further devoid of fluorescence removal.

In some embodiments, the optical spectrum of the sample is obtained during an inspection of the gas turbine engine using a spectroscopy apparatus.

In some embodiments, each of the one or more pre-determined reference values corresponding to the characteristic parameter is based on a previously obtained normalised optical spectrum of a reference sample.

In some embodiments, the optical spectrum of the sample includes a Raman spectrum, an infrared spectrum, or a terahertz spectrum.

In some embodiments, the method is performed in-situ during the inspection of the gas turbine engine.

In some embodiments, the at least one compound in the sample includes an engine oil or an inhibitor fluid.

In some embodiments, the characteristic parameter includes a spectral density of the normalised optical spectrum.

In some embodiments, the spectral density of the normalised optical spectrum is calculated as the area under a curve of the normalised optical spectrum.

In some embodiments, the characteristic parameter includes a function of spectrum intensity and wavenumber of a curve of the normalised optical spectrum.

As noted elsewhere herein, the present invention may relate to the maintenance of a gas turbine engine. Such a gas turbine engine may comprise an engine core comprising a turbine, a combustor, a compressor, and a core shaft connecting the turbine to the compressor. Such a gas turbine engine may comprise a fan (having fan blades) located upstream of the engine core. The skilled person will appreciate that except where mutually exclusive, a feature or parameter described in relation to any one of the above aspects may be applied to any other aspect. Furthermore, except where mutually exclusive, any feature or parameter described herein may be applied to any aspect and/or combined with any other feature or parameter described herein.

### BRIEF DESCRIPTION OF THE DRAWINGS

Embodiments will now be described by way of example only with reference to the accompanying drawings, in which:
**FIG. 1** is a sectional side view of a gas turbine engine;
**FIG. 2** is a flowchart depicting various steps of the method of performing spectral analysis on a sample;
**FIG. 3** is a block diagram depicting obtainment of an optical spectrum;
**FIG. 4** illustrates a graph depicting an example normalised optical spectrum of a sample;
**FIG. 5A** illustrates a graph depicting a normalised optical spectrum of a first reference sample;
**FIG. 5B** illustrates a graph depicting a normalised optical spectrum of a second reference sample;
**FIG. 5C** illustrates a graph depicting a normalised optical spectrum of a third reference sample; and
**FIG. 6** is a flowchart depicting various steps of a process for inspecting a gas turbine engine and analysing a sample found therein.

The following table lists the reference numerals used in the drawings with the features to which they refer:

| Ref no. | Feature | Figure |
|---|---|---|
| 9 | Principal rotational axis | 1 |
| 10 | Gas turbine engine | 1 3 |
| 11 | Engine core | 1 |
| 12 | Air intake | 1 |
| 14 | Low pressure compressor (LPC) | 1 |
| 15 | High pressure compressor (HPC) | 1 |
| 16 | Combustion equipment | 1 |
| 17 | High pressure turbine (HPT) | 1 |
| 18 | Bypass exhaust nozzle | 1 |
| 19 | Low pressure turbine (LPT) | 1 |
| 20 | Core exhaust nozzle | 1 |
| 21 | Nacelle | 1 |
| 22 | Bypass duct | 1 |
| 23 | Fan | 1 |
| 26 | Shaft | 1 |
| 27 | Shaft | 1 |
| 30 | Epicyclic gearbox | 1 |
| 100 | Method | 2 |
| 102 | Step | 2 |
| 104 | Step | 2 |
| 106 | Step | 2 |
| 108 | Step | 2 |
| 110 | Step | 2 |
| 112 | Step | 2 |
| 220 | Sample | 3 |
| 230 | Spectroscopy apparatus | 3 |
| 240 | Optical spectrum | 34 |
| 300 | Graph | 4 |
| 301 | Normalised optical spectrum | 4 |
| 305 | Curve | 4 |
| 320 | First wavenumber | 4 |
| 330 | Second wavenumber | 4 |
| 340 | Region of Interest | 4 |
| 400 | Graph | 5A |
| 401 | Normalised optical spectrum | 5A |
| 410 | Graph | 5B |
| 411 | Normalised optical spectrum | 5B |
| 420 | Graph | 5C |
| 421 | Normalised optical spectrum | 5C |
| 500 | Process | 6 |
| 502 | Block | 6 |
| 504 | Block | 6 |
| 506 | Block | 6 |
| 508 | Block | 6 |
| 510 | Block | 6 |
| 512 | Block | 6 |
| A | Core air flow | 1 |
| B | Bypass air flow | 1 |

### DETAILED DESCRIPTION

Aspects and embodiments of the present invention will now be discussed with reference to the accompanying figures. Further aspects and embodiments will be apparent to those skilled in the art.

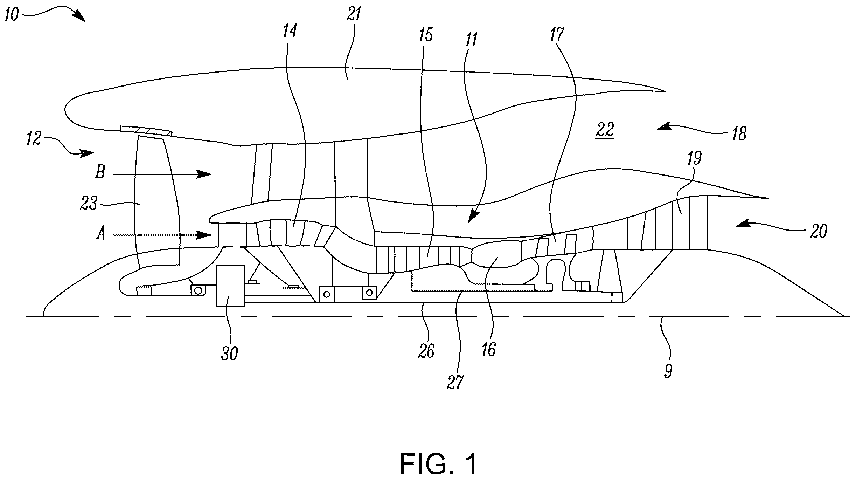

**FIG. 1** illustrates a gas turbine engine 10 having a principal rotational axis 9. The engine 10 includes an air intake 12 and a propulsive fan 23 that generates two airflows: a core airflow A and a bypass airflow B. The gas turbine engine 10 includes a core 11 that receives the core airflow A. The engine core 11 includes, in axial flow series, a low pressure compressor 14, a high pressure compressor 15, combustion equipment 16, a high pressure turbine 17, a low pressure turbine 19, and a core exhaust nozzle 20. A nacelle 21 surrounds the gas turbine engine 10 and defines a bypass duct 22 and a bypass exhaust nozzle 18. The bypass airflow B flows through the bypass duct 22. The fan 23 is attached to and driven by the low pressure turbine 19 via a shaft 26 and an epicyclic gearbox 30.

In use, the core airflow A is accelerated and compressed by the low pressure compressor 14 and directed into the high pressure compressor 15 where further compression takes place. The compressed air exhausted from the high pressure compressor 15 is directed into the combustion equipment 16 where it is mixed with fuel and the mixture is combusted. The resultant hot combustion products then expand through, and thereby drive, the high pressure and low pressure turbines 17, 19 before being exhausted through the core exhaust nozzle 20 to provide some propulsive thrust. The high pressure turbine 17 drives the high pressure compressor 15 by a suitable interconnecting shaft 27. The fan 23 generally provides the majority of the propulsive thrust. The epicyclic gearbox 30 is a reduction gearbox.

Note that the terms "low pressure turbine" and "low pressure compressor", as used herein, may be taken to mean the lowest pressure turbine stages and lowest pressure compressor stages (i.e., not including the fan 23), respectively, and/or the turbine and compressor stages that are connected together by the interconnecting shaft 26 with the lowest rotational speed in the engine 10 (i.e., not including the gearbox output shaft that drives the fan 23). In some literature, the "low pressure turbine" and "low pressure compressor" referred to herein may alternatively be known as the "intermediate pressure turbine" and "intermediate pressure compressor". Where such alternative nomenclature is used, the fan 23 may be referred to as a first, or lowest pressure, compression stage.

Other gas turbine engines to which the method of the present invention may be applied may have alternative configurations. For example, such engines may have an alternative number of compressors and/or turbines and/or an alternative number of interconnecting shafts. By way of further example, the gas turbine engine 10 shown in Figure 1 has a split flow nozzle 18, 20 meaning that the flow through the bypass duct 22 has its own nozzle 18 that is separate to and radially outside the core exhaust nozzle 20. However, this is not limiting, and any aspect of the present invention may also apply to engines in which the flow through the bypass duct 22 and the flow through the core 11 are mixed, or combined, before (or upstream of) a single nozzle, which may be referred to as a mixed flow nozzle. One or both nozzles (whether mixed or split flow) may have a fixed or variable area. Whilst the described example relates to a turbofan engine, the invention may apply, for example, to any type of gas turbine engine, such as an open rotor (in which the fan stage is not surrounded by a nacelle), or turboprop engine, for example. In some arrangements, the gas turbine engine 10 may not comprise a gearbox 30.

The geometry of the gas turbine engine 10, and components thereof, is defined by a conventional axis system, comprising an axial direction (which is aligned with the rotational axis 9), a radial direction (in the bottom-to-top direction in Figure 1), and a circumferential direction (perpendicular to the page in the Figure 1 view). The axial, radial, and circumferential directions are mutually perpendicular.

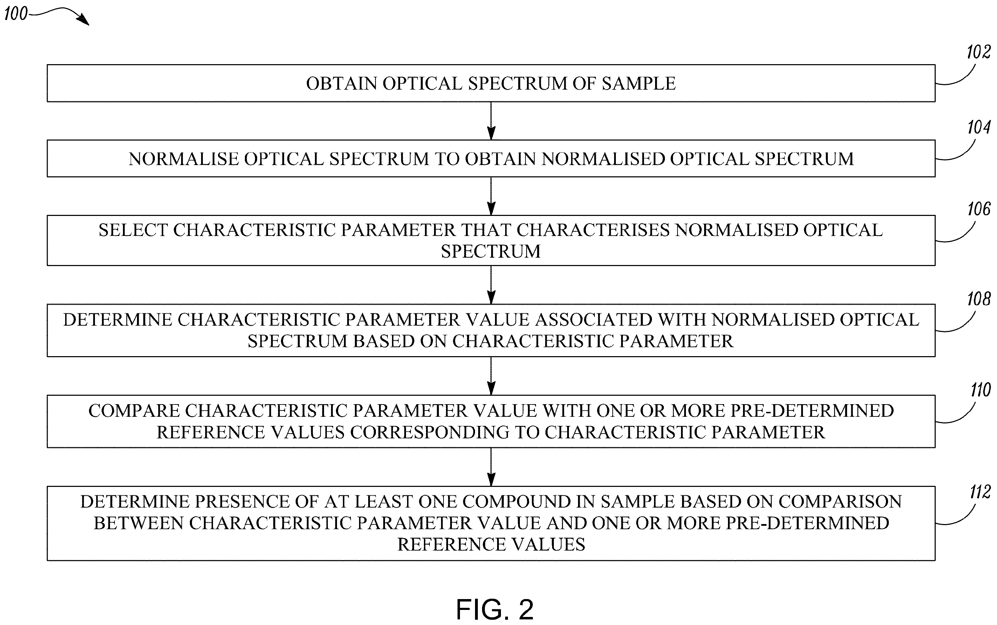

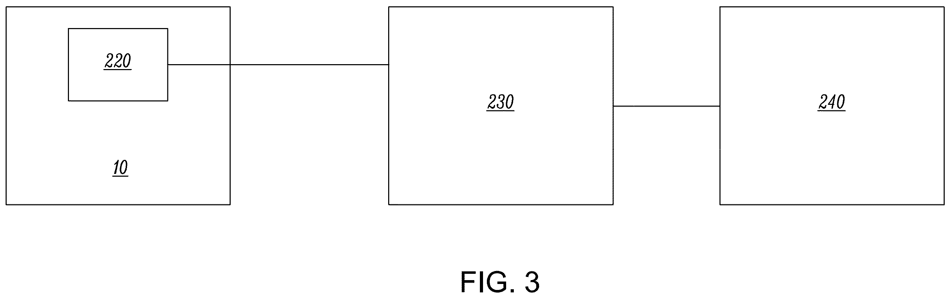

**FIG. 2** shows a flowchart depicting various steps of a method 100 of performing spectral analysis on a sample identified within a gas turbine engine (e.g., the gas turbine engine 10 of FIG. 1). The method 100 will be discussed with additional reference to FIGS. 3 to 5C.

At step 102, the method 100 includes obtaining an optical spectrum of the sample.

The sample may be a portion of a substance that is identified within the gas turbine engine. The sample may be representative of the substance. The sample may be a fluid, a semi-solid, or a solid sample.

As used herein, the term "optical spectrum" refers to data representing an intensity of light scattered off an object. The data of an optical spectrum may be generally represented in a graphical representation. Some optical spectra may be intensity versus wavelength spectra, while others may be intensity versus wavenumber spectra. Embodiments of the present invention may be suitable for optical spectra of various different types. In some embodiments, the optical spectrum of the sample may include a Raman spectrum, an infrared spectrum, or a terahertz spectrum.

The optical spectrum of the sample may be obtained using a suitable spectroscopy apparatus. The spectroscopy apparatus may include any type of instrumentation, including, but not limited to, spectroscopes, spectrographs, spectrophotometers, that can scan and report a portion of the electromagnetic radiation spectrum (i.e., microwave, far infrared, midinfrared, near infrared, visible, ultraviolet, x-ray, terahertz (THz), etc). In some embodiments, the spectroscopy apparatus may include a miniature spectroscopy probe.

In some embodiments, the optical spectrum of the sample may be obtained during an inspection of the gas turbine engine using the spectroscopy apparatus. Referring to FIGS. 2 and 3, for example, the method 100 may include obtaining an optical spectrum 240 of a sample 220 during an inspection of the gas turbine engine 10 using a spectroscopy apparatus 230. The spectroscopy apparatus 230 may generate the optical spectrum 240 of the sample 220.

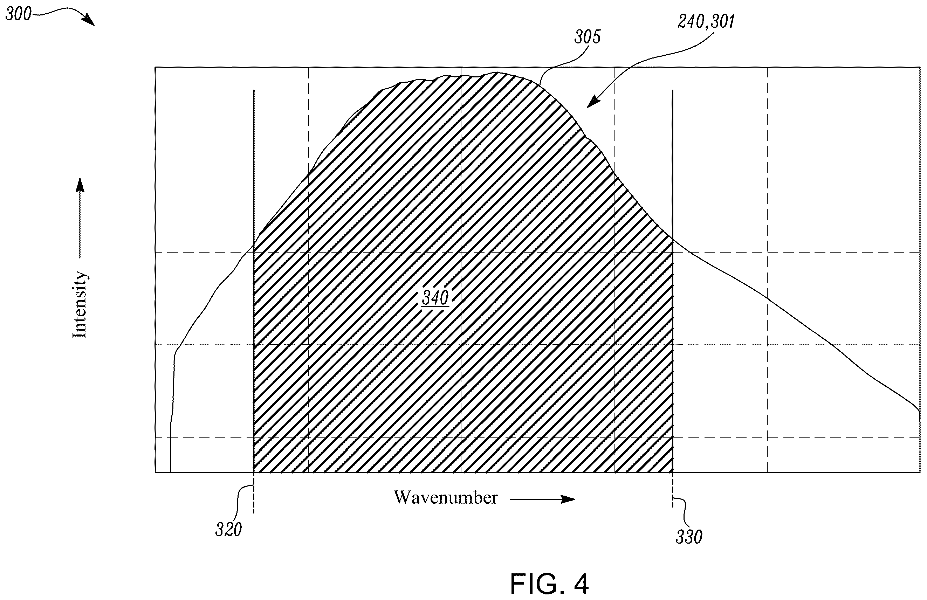

An example of the optical spectrum 240 is shown in FIG. 4. Specifically, FIG. 4 shows a graph 300 including a curve 305 depicting a variation of intensity with respect to wavenumber of the optical spectrum 240. The horizontal axis represents the wavenumber, and the vertical axis represents a magnitude of the spectral intensity of the optical spectrum 240.

At step 104, the method 100 further includes normalising the optical spectrum to obtain a normalised optical spectrum.

The normalised optical spectrum may be obtained by scaling the optical spectrum with a scaling factor (in the spectral intensity axis). In one example, to obtain the normalised optical spectrum, the optical spectrum may be scaled such that the peak intensity in the optical spectrum is transformed into a pre-defined peak intensity. Other normalising methods may be alternatively employed.

The normalisation process may enable comparison of optical spectra obtained from different samples or under different conditions. Further, the normalisation process may help mitigate common effects of varying intensities between measurements, which can arise due to factors such as sample preparation, instrumental drift, or changes in experimental conditions. By normalising the optical spectrum, the data of the optical spectrum may become more comparable to previously obtained spectral data.

**FIG. 4** shows an example normalised optical spectrum 301 obtained from the optical spectrum 240 of **FIG. 3****.** For explanatory purposes, the curve 305 of FIG. 4 is assumed to depict both the optical spectrum 240 and the normalised optical spectrum 301.

In some embodiments, the method 100 may further include determining a region of interest across the optical spectrum. The region of interest may be determined by a trialand-error method. Furthermore, normalising the optical spectrum (at step 104) may include normalising the optical spectrum defined in the region of interest. In such embodiments, the normalised optical spectrum may include a normalised portion of the optical spectrum defined in the region of interest.

The region of interest may extend from a first wavenumber to a second wavenumber that is greater than the first wavenumber. Alternatively, in some other embodiments, the region of interest may extend from a first wavelength to a second wavelength that is greater than the first wavelength.

Referring to FIG. 4, for example, the method 100 may include determining a region of interest 340 (cross-hatched in FIG. 4) across the optical spectrum 240, and the step of normalising the optical spectrum 240 may be performed for a portion of the optical spectrum 240 disposed in the region of interest 340. In FIG. 4, the region of interest 340 extends from a first wavenumber 320 to a second wavenumber 330.

In some embodiments, the method 100 may further include processing the optical spectrum of the sample prior to normalising the optical spectrum. For example, the optical spectrum may be processed to remove anomalies, such as cosmic rays, and perform basic background noise removal. Such processing of the optical spectrum of the sample may have low computational requirements.

The processing may be devoid of deconvolution processing. Deconvolution processing may be a computationally expensive process which includes filtering a signal to recreate the signal as it existed before the convolution took place. The processing of the optical spectrum of the sample may be further devoid of fluorescence removal. Fluorescence removal may be a process of removing anomalies in a signal due to a fluorescence effect. In contrast to conventional spectral analysis techniques, which typically employ deconvolution processing and/or fluorescence removal, the method 100 may be devoid therefrom.

At step 106, the method 100 further includes selecting a characteristic parameter that characterises the normalised optical spectrum.

The characteristic parameter may be any suitable parameter that can transform the normalised optical spectrum into a single value. The characteristic parameter of a normalised optical spectrum may be used to associate a value with the normalised optical spectrum. In some examples, the characteristic parameter may characterise the overall shape of a curve of the normalised optical spectrum.

In some embodiments, the characteristic parameter may include a spectral density of the normalised optical spectrum (also known as Normalised Spectral Density (NSD)). The spectral density may be calculated as the area under the curve of the normalised optical spectrum.

In some embodiments, the characteristic parameter may include a function of spectrum intensity and wavenumber of a curve of the normalised optical spectrum. In other words, the characteristic parameter may be calculated as a function of two variables, namely, the spectrum intensity and the wavenumber of the normalised optical spectrum.

At step 108, the method 100 further includes determining a characteristic parameter value associated with the normalised optical spectrum based on the characteristic parameter. In other words, at step 108, the method 100 may include calculating the characteristic parameter value for the normalised optical spectrum based on the selected characteristic parameter.

Referring to FIG. 4, for example, the selected characteristic parameter value may be the spectral density. The spectral density of the normalised optical spectrum 301 may be calculated as the area under the curve 305 of the normalised optical spectrum 301 in the region of interest 340.

At step 110, the method 100 further includes comparing the characteristic parameter value with one or more pre-determined reference values corresponding to the characteristic parameter.

Each of the one or more pre-determined reference values corresponding to the characteristic parameter may be based on a previously obtained normalised optical spectrum of a reference sample or a class of reference samples. A database storing previously obtained characteristic parameter values associated with reference samples may be constructed prior to performing the method 100. The database may be queried to compare the determined characteristic parameter value (at step 108) with the previously obtained characteristic parameter values.

At step 112, the method 100 further determining a presence of at least one compound in the sample based on the comparison between the characteristic parameter value and the one or more pre-determined reference values.

The method 100 may facilitate determining the at least one compound in the sample in a computationally efficient manner. The method 100 may utilise the optical spectrum without performing computationally expensive pre-processing operations (e.g., deconvolution, fluorescence removal, etc.) on the optical spectrum, which are typically performed in conventional spectral analysis techniques. The method 100 may be conveniently performed, for example, using edge computing devices, such as handheld computing devices near the gas turbine engine due to the low computational complexity compared to conventional spectral analysis techniques. Moreover, the method 100 may utilise the fluorescence background in the optical spectrum to determine the at least one compound, in contrast to conventional spectral analysis techniques that consider fluorescence as noise.

The method 100 may also facilitate inspection and maintenance of the gas turbine engine. For example, the method 100 may simplify decision-making during a maintenance procedure based on the determined presence of the at least one compound in the sample. By way of example, the method 100 may facilitate a decision to further strip the gas turbine engine or continue inspection without further stripping the gas turbine engine.

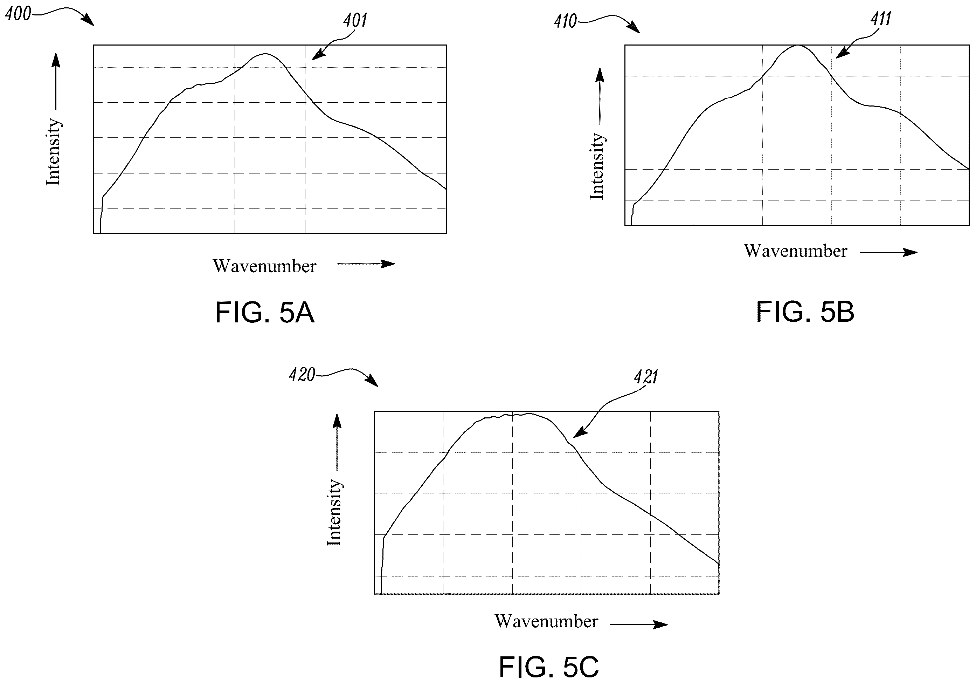

In some embodiments, the at least one compound in the sample may include an engine oil or an inhibitor fluid. FIGS. 5A, 5B, and 5C illustrate graphs 400, 410, and 420, respectively, showing examples of previously obtained normalised optical spectra of three different references samples. A database may store pre-determined reference values corresponding to the previously obtained normalised optical spectra, which may be used for comparison in embodiments herein.

A first normalised optical spectrum 401 (depicted in **FIG. 5A**) may be of a first reference substance sample. For example, the first normalised optical spectrum 401 may be of a sample of the engine oil of the gas turbine engine.

A second normalised optical spectrum 411 (depicted in **FIG. 5B**) may be of a second reference substance sample. For example, the second normalised optical spectrum 411 may be of a sample of the inhibitor fluid used in the gas turbine engine.

A third normalised optical spectrum 421 (depicted in **FIG. 5C**) may be of a third reference substance sample. For example, the third normalised optical spectrum 421 may be of a sample containing a mixture of 60% of the engine oil and 40% of the inhibitor fluid.

As an example, if the characteristic parameter is selected as the spectral density, the characteristic parameter value for the first normalised optical spectrum 401 may be V1, the characteristic parameter value for the second normalised optical spectrum 411 may be V2, and the characteristic parameter value for the third normalised optical spectrum 421 may be V3.

If a smear of substance is identified within the gas turbine engine, a sample of the smear may be collected, and the method 100 may be performed on the smear sample. If the characteristic parameter value for the smear sample is determined to be a value close to V2, it may be determined that the smear is of the inhibitor fluid. However, if the characteristic parameter value of the smear sample is determined to be a value close to V1 or V2, it may be determined that the smear identified within the gas turbine engine contains the engine oil. If the smear identified within the gas turbine engine contains the engine oil, the gas turbine engine may need to be further stripped to determine the cause of engine oil leak. In contrast, if the smear identified within the gas turbine engine is free of the engine oil, the maintenance may be continued without further stripping of the gas turbine engine. In this way, the method 100 may promote stripping of the gas turbine engine when required.

In some embodiments, the method 100 is performed in-situ during the inspection of the gas turbine engine. In other words, the method 100 may be carried out during the inspection of the gas turbine while the gas turbine engine is attached to an aircraft. Based on the result of the method 100, i.e., presence of the at least one compound in the sample, the gas turbine engine may be decoupled from the aircraft, if required. In this way, the method 100 may reduce costs associated with unnecessarily decoupling of the gas turbine engine from the aircraft.

Optionally, in some embodiments, the method 100 may further include applying an additive to the sample. The additive may be configured to enhance or alter the spectral response of the sample. This may further improve the performance of the method 100.

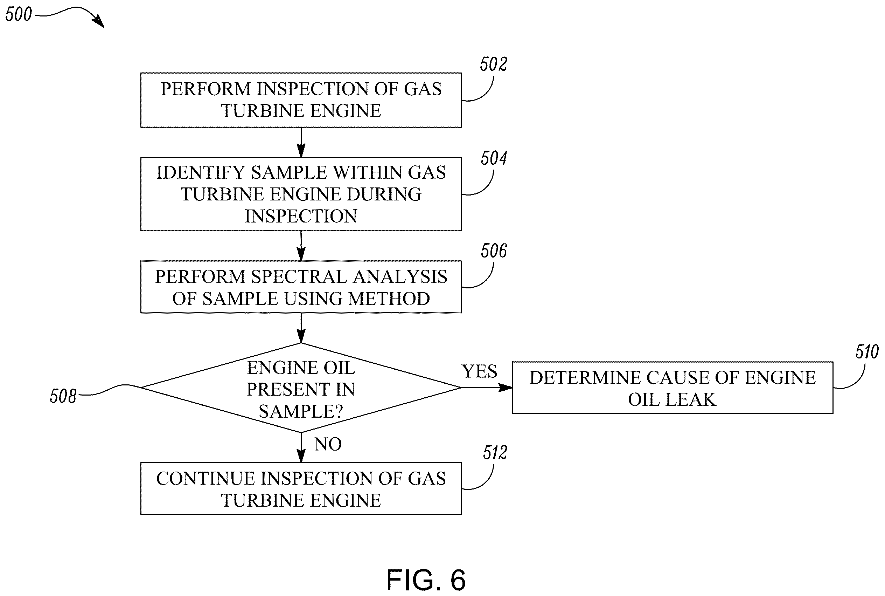

**FIG. 6** is a flowchart depicting various steps of a process 500 for a gas turbine engine incorporating the method 100 of FIG. 2.

At block 502, the process 500 includes performing inspection of a gas turbine engine. The inspection of the gas turbine engine may be performed during a routine maintenance procedure or an overhauling procedure of the gas turbine engine.

At block 504, the process 500 includes identifying a sample within the gas turbine engine during the inspection.

At block 506, the process 500 includes performing spectral analysis of the sample using the method 100 of FIG. 2. In other words, at block 506, the process 500 includes determining presence of at least one compound in the sample based on the comparison between a determined characteristic parameter value and one or more pre-determined reference values using the method 100.

At block 508, the process 500 includes determining whether an engine oil present in the sample based on the result of block 506. If the engine oil is present in the sample, the process 500 proceeds to block 510. If the engine oil is not present in the sample, the process 500 proceeds to block 512.

At block 510, the process 500 includes determining the cause of engine oil leak. In some examples, at block 510, the process 500 may include further stripping the gas turbine engine to determine the cause of the engine oil leak.

At block 512, the process 500 includes continuing the inspection of the gas turbine engine. For example, the inspection of the gas turbine engine may continue without further stripping of the gas turbine engine.

It will be understood that the invention is not limited to the embodiments above-described and various modifications and improvements can be made without departing from the concepts described herein. Except where mutually exclusive, any of the features may be employed separately or in combination with any other features and the invention extends to and includes all combinations and sub-combinations of one or more features described herein.

Various examples have been described, each of which comprise one or more combinations of features. It will be appreciated by those skilled in the art that, except where clearly mutually exclusive, any of the features may be employed separately or in combination with any other features and the invention extends to and includes all combinations and sub-combinations of one or more features described herein.

## Claims

1. A method (100) of performing spectral analysis on a sample (220) identified within a gas turbine engine (10), the method (100) comprising the steps of:
obtaining (102) an optical spectrum (240) of the sample (220);
normalising (104) the optical spectrum (240) to obtain a normalised optical spectrum (301);
selecting (106) a characteristic parameter that characterises the normalised optical spectrum (301);
determining (108) a characteristic parameter value associated with the normalised optical spectrum (301) based on the characteristic parameter;
comparing (110) the characteristic parameter value with one or more pre-determined reference values corresponding to the characteristic parameter; and
determining (112) a presence of at least one compound in the sample (220) based on the comparison between the characteristic parameter value and the one or more pre-determined reference values.

2. The method (100) of claim 1, further comprising determining a region of interest (340) across the optical spectrum (240) and normalising the optical spectrum (240) comprises normalising the optical spectrum (240) defined in the region of interest (340).

3. The method (100) of claim 2, wherein the region of interest (340) extends from a first wavenumber (320) to a second wavenumber (330) that is greater than the first wavenumber (320).

4. The method (100) of any preceding claim, further comprising processing the optical spectrum (240) of the sample prior to normalising the optical spectrum (240), wherein the processing is devoid of deconvolution processing.

5. The method (100) of claim 4, wherein the processing of the optical spectrum (240) of the sample (220) is further devoid of fluorescence removal.

6. The method (100) of any preceding claim, wherein the optical spectrum (240) of the sample (220) is obtained during an inspection of the gas turbine engine (10) using a spectroscopy apparatus (230).

7. The method (100) of any preceding claim, wherein each of the one or more pre-determined reference values corresponding to the characteristic parameter is based on a previously obtained normalised optical spectrum of a reference sample.

8. The method (100) of any preceding claim, wherein the optical spectrum (240) of the sample (220) comprises a Raman spectrum, an infrared spectrum, or a terahertz spectrum.

9. The method (100) of any preceding claim, wherein the method (100) is performed in-situ during the inspection of the gas turbine engine (10).

10. The method (100) of any preceding claim, wherein the at least one compound in the sample (220) comprises an engine oil or an inhibitor fluid.

11. The method (100) of any preceding claim, wherein the characteristic parameter comprises a spectral density of the normalised optical spectrum (301).

12. The method (100) of claim 11, wherein the spectral density is calculated as the area under a curve of the normalised optical spectrum (301).

13. The method (100) of any one of claims 1 to 10, wherein the characteristic parameter comprises a function of spectrum intensity and wavenumber of a curve of the normalised optical spectrum (301).
